# EUROPEAN PATENT SPECIFICATION

(11) **EP 2 832 744 B1**
(45) Date of publication and mention of the grant of the patent: **19.09.2018**
(21) Application number: 13769065.7
(22) Date of filing: 29.03.2013
(51) Int. Cl.: C07K 7/56, A61K 38/12, A61P 31/10, C07K 7/64, C07K 7/06

(54) **HYDRATE OF CYCLOPEPTIDE COMPOUND AS WELL AS PREPARATION METHOD AND USE THEREOF**
HYDRAT AUS EINER CYCLOPEPTIDVERBINDUNG SOWIE HERSTELLUNGSVERFAHREN UND VERWENDUNG DAVON
HYDRATE D'UN COMPOSÉ CYCLOPEPTIDIQUE, SON PROCÉDÉ DE PRÉPARATION ET SON UTILISATION

(30) Priority: 30.03.2012 CN 201210090377
(43) Date of publication of application: 04.02.2015
(73) Proprietor: Shanghai Techwell Biopharmaceutical Co., Ltd, Shanghai 201108 (CN)
(72) Inventor: LIU, Shidong, Shanghai 201108 (CN); ZHANG, Zhaoli, Shanghai 201108 (CN); WANG, Xiusheng, Shanghai 201108 (CN); ZHANG, Xiao, Shanghai 201108 (CN); TANG, Zhijun, Shanghai 201108 (CN); JI, Xiaoming, Shanghai 201108 (CN)
(74) Representative: Germinario, Claudio
(86) International application number: PCT/CN2013/073516
(87) International publication number: WO 2013/143501

(56) References cited:
- EP-A2- 0 462 531
- EP-B1- 0 788 511
- WO-A1-2004/014879
- WO-A1-2012/136498
- WO-A1-2013/034670
- CN-A- 1 059 729
- CN-A- 1 168 675
- CN-A- 102 627 689
- STEFAN C SCHNEID ET AL: "Optimization of the Secondary Drying Step in Freeze Drying Using TDLAS Technology", AAPS PHARMSCITECH, SPRINGER NEW YORK LLC, US, vol. 12, no. 1, 26 February 2011 (2011-02-26), pages 379-387, XP019891319, ISSN: 1530-9932, DOI: 10.1208/S12249-011-9600-7
- MASAKI TOMISHIMA ET AL: "FK463, a Novel Water-soluble Echinocandin Lipopeptide: Synthesis and Antifungal Activity", JOURNAL OF ANTIBIOTICS, JAPAN ANTIBIOTICS RESEARCH ASSOCIATION, TOKYO, JP, vol. 52, no. 7, 1 July 1999 (1999-07-01), pages 674-676, XP008157956, ISSN: 0021-8820, DOI: 10.7164/ANTIBIOTICS.52.674 [retrieved on 2008-09-19]

## Description

### Technical field

The present description relates to a pharmaceutical composition, and more particularly relates to a pharmaceutical composition comprising a hydrate of cyclopeptide compound having good stability as well as the preparation method and use thereof.

### Background

Fungal infection has become the leading cause for high morbidity and mortality in immunodeficient patients. During the past 20 years, the incidence of fungal infection increased significantly. People at high-risk of fungal infections includes critical patients, surgical patients and those patients suffering from HIV infection, leukemia and other tumors. Patients with organ transplant are also at high risk of fungal infection.

Echinocandins, as a new class of antifungal agents, exhibit good effects in the treatment of infections caused by Candida or Aspergillus. Caspofungin and Micafungin are the representatives of such medicaments. Echinocandins inhibit fungus by suppressing the formation of 1,3-β glycosidic bond, so as to reduce the harm to human body, and reduce the side effects while remaiming high efficiency. Therefore, they are safer in use than traditional antifungal agents.

FK463 (sodium Micafungin) is the compound of formula II (R is a sodium ion), which is developed by Japan Fujisawa Toyama Co., Ltd, Takaoka Plant under the trade name Mycamine, and currently sold in several countries as antifungal agent for intravenous administration. It is obtained by cutting the side-chain of FR901379 as precursor (compound of Formula III, R is a sodium ion or a hydrogen ion) by enzyme, thus forming FR179642 (compound of Formula I, R is a hydrogen or a sodium ion) (see U.S. Patent No. US5376634, EP0431350 and Chinese patent CN1161462C for specific methods), and then chemically modifying FR179642 (see Patent Publication WO9611210, WO9857923, WO2004014879 for specific preparation and purification methods).

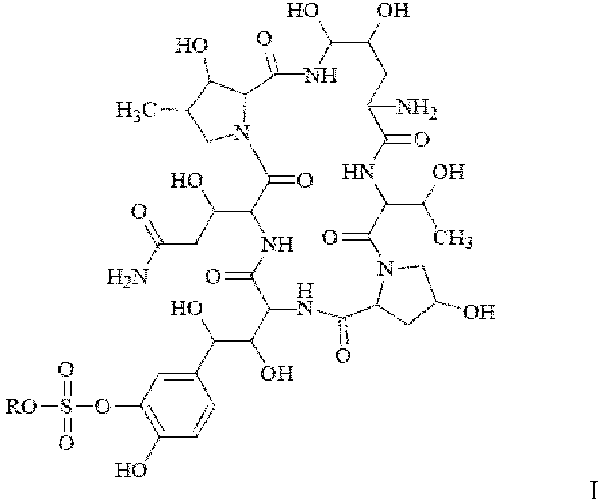

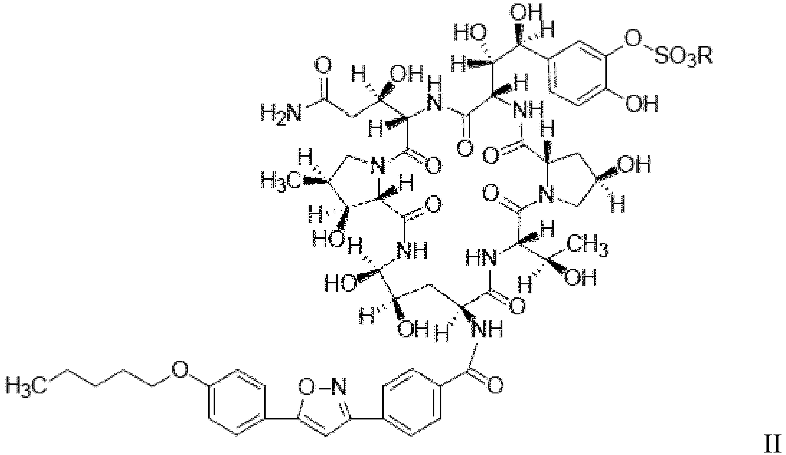

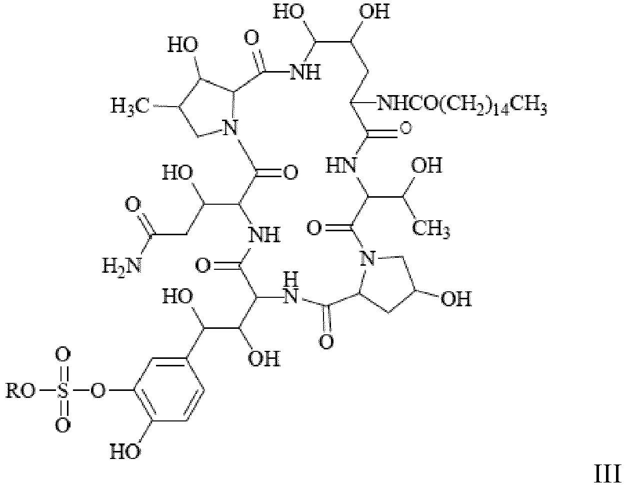

Specific scheme is shown as follows:

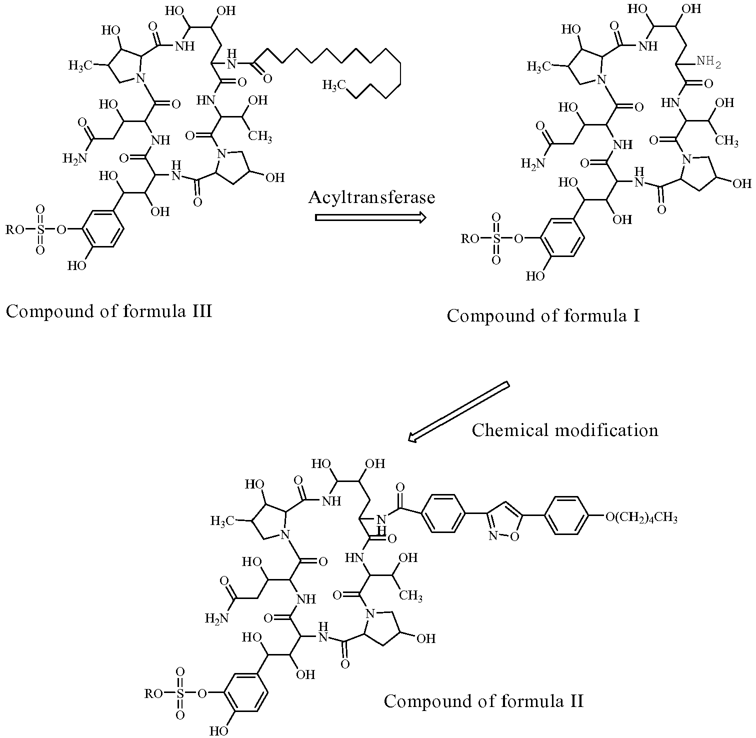

As well-known in the art, the drug stability is closely related to the moisture content. It is reported in literatures and books (e.g., "*Pharmaceutics*") relating to drug stability that water is the medium for chemical reaction, and after water is absorbed by a drug in solid form, a liquid film will form on its surface, and hydrolysis or oxidative decomposition reaction will occur in the film. Trace amount of water can accelerate the decomposition of unstable drugs. Moisture content of raw medicine, such as ampicillin, should be controlled at a relatively low level, generally about 1%. The higher the moisture content, the faster decomposition goes.

After extensive researches, the inventors have found that the moisture content of compound of formula I has an important effect on the stability of the compound. Even more surprisingly, the inventors have found that high moisture content will effectively improve the stability of the compound of formula I, instead of accelerating the decomposition of the compound and deteriorating the stability of the compound. When the moisture content of the compound of formula I is less than 8%, stability of the compound is significantly reduced, as described above. The inventors have also found that the stability of compound of Formula I is less related with the types of crystalline form, while the moisture content is critical to the stability of the compound. Such findings are unexpected, and concluded by the inventor through a great deal of experiments.

Therefore, there is an urgent need in the art to provide a hydrate of the compound of formula I, which possesses excellent stability and is more suitable for transportation and preservation.

### SUMMARY OF THE INVENTION

The invention is defined in the claims.

In one aspect, a hydrate of the compound of formula I is provided by the present invention, and R represents H or a cation capable of forming a pharmaceutically acceptable salt, wherein the mass percent of water in the hydrate is between 8.0% and 30%, and R represents preferably H, a sodium ion or a diisopropylethylamine ion;

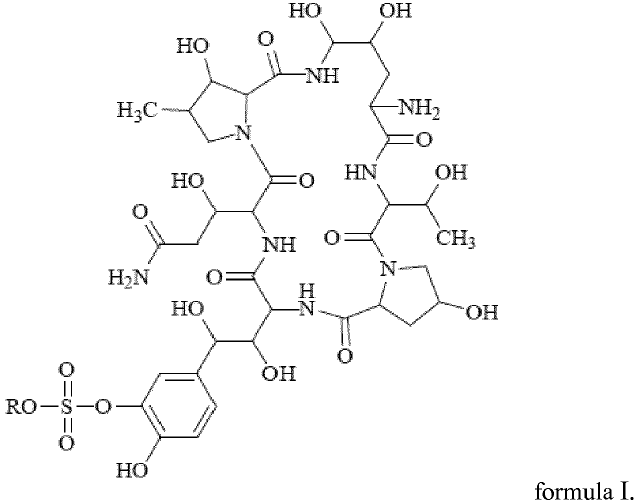

The mass percent of water is 8-30%.

In a further embodiment, the mass percent of water is 9.5-28.0%.

In another embodiment, the hydrate is prepared through the following steps:
(a) dissolving the compound of formula I into water or aqueous water-miscible organic solvent (i), and controlling pH of the solution comprising the compound of formula I;
(b) obtaining the hydrate comprising the compound of formula I by reducing the temperature and / or adding water-miscible organic solvent (i);
(c) obtaining the hydrate by centrifuging or filtrating;
(d) vacuum-drying the hydrate obtained in step (c) and controlling the mass percent of water in the solid.

In a further embodiment, said organic solvent (i) is selected from C1-C4 lower alcohol.

In a further embodiment, the lower alcohol is one or more selected from the group consisting of methanol, ethanol, n-propanol, and isopropanol.

In a further embodiment, pH of the solution comprising the compound of formula I is controlled at 2.0-5.0; in a further embodiment, pH of the solution comprising the compound of formula I is controlled at 3.5-4.5.

In a further embodiment, the mass percent of water in the solid is controlled at 8% - 30%.

In a further embodiment, the mass percent of water in the solid is controlled at 9.5% - 28.0%.

In another aspect, a hydrate of the compound of formula I is provided by the present invention, including the following steps:
(a) dissolving the compound of formula I into water or aqueous water-miscible organic solvent (i), and controlling pH of the solution comprising the compound of formula I;
(b) obtaining the hydrate comprising the compound of formula I by reducing the temperature and / or adding water-miscible organic solvent (i);
(c) obtaining the hydrate by centrifuging or filtrating;
(d) vacuum-drying the hydrate obtained in step (c) and controlling the mass percent of water in the hydrate.

In a further embodiment, said organic solvent (i) is selected from C1-C4 lower alcohol.

In a further embodiment, the lower alcohol is one or more selected from the group consisting of methanol, ethanol, n-propanol, and isopropanol.

In a further embodiment, pH of the solution comprising the compound of formula I is controlled at 2.0-5.0; in a further embodiment, pH of the solution comprising the compound of formula I is controlled at 3.5-4.5.

In a further embodiment, the mass percent of water in the solid is controlled at 8% - 30%.

In a further embodiment, the mass percent of water in the solid is controlled at 9.5% - 28.0%.

In another aspect, uses of the hydrate for preparing the compound of formula II are provided by the invention

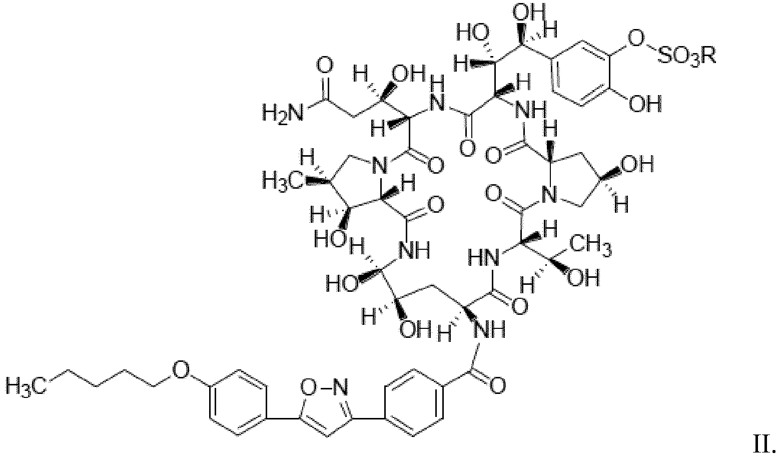

In another aspect, uses of the hydrate for preparing medicaments for treating fungal infections are provided by the invention.

In another aspect, a pharmaceutical composition comprising the above hydrate and a pharmaceutically acceptable carrier is provided by the description.

In another aspect, a preparation method for the pharmaceutical composition is provided by the invention, including mixing the hydrate with pharmaceutically acceptable carrier, so as to obtain the pharmaceutical composition.

In another aspect, the hydrate prepared the above methods is provided by the invention.

### Brief description of the Drawings

Figure 1 is the HPLC pattern for hydrate D prepared in Example 2 after placed at 25°C for 6 months.
Figure 2 is the HPLC pattern for hydrate Y prepared in Example 6 after placed at 25°C for 6 months.

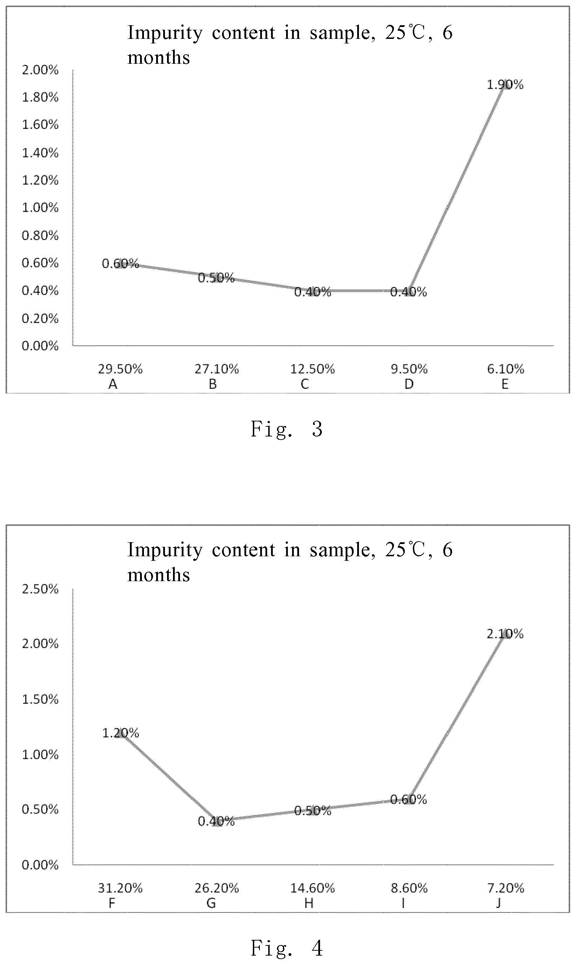

Figure 3 shows the amounts of impurities in the sample for hydrate A, B, C, D and E after placed at 25°C for 6 months.
Figure 4 shows the amounts of impurities in the sample for hydrate F, G, H, I and J after placed at 25°C for 6 months.

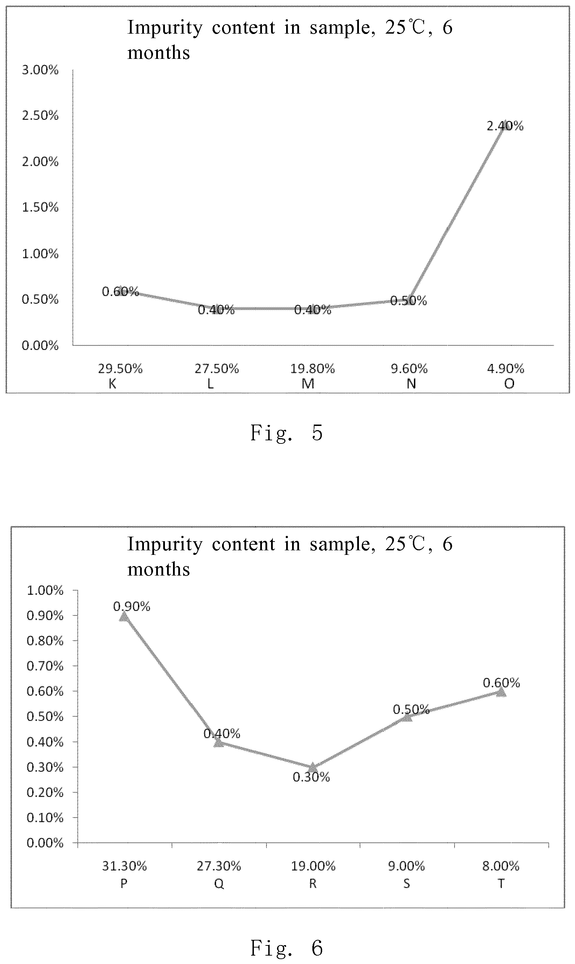

Figure 5 shows the amounts of impurities in the sample for hydrate K, L, M, N and O after placed at 25°C for 6 months.
Figure 6 shows the amounts of impurities in the sample for hydrate P, Q, R, S and T after placed at 25°C for 6 months.

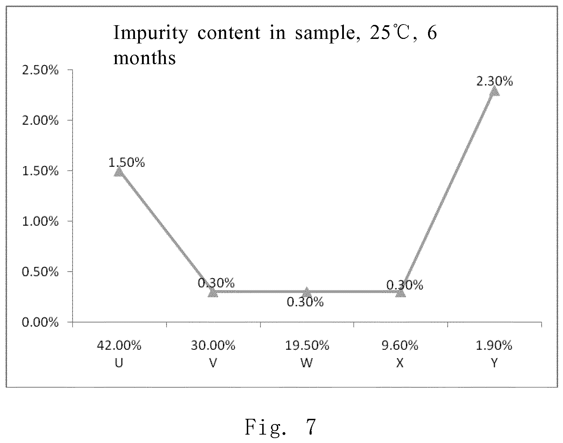

Figure 7 shows the amounts of impurities in the sample for hydrate U, V, W, X and Y after placed at 25°C for 6 months.

### The mode for carrying out the invention

After extensive researches, the inventors discovered that hydrates of the compound of formula I can be obtained by dissolving the compound into water or mixture solution of water-miscible lower alcohols, maintaining the solubility of the solution comprising the compound of formula I around saturated and controlling pH value of the solution within specified range. Even more importantly, the hydrate formed from the compound of formula I comprises water, and the inventors have discovered, based on extensive researches, that the moisture content in the hydrate of compound of formula I will have important effects on the stability of hydrate. For the preparation method of the invention, a great deal of intensive researching works have been performed on screening solvents for crystallization, and it is found that crystals with excellent morphology can be formed from the compound of formula I by crystallizing the compound in methanol, ethanol, n-propanol, isopropanol or the mixture solution thereof, and the compound of formula I with excellent stability can be obtained by controlling the moisture content within certain range. When crystallizing the compound in a solvent such as acetone, acetonitrile, ethyl acetate, amorphous precipitate with poor stability will be formed from the compound of formula I, and that is the reason for difference in stability between amorphous solids and crystals substance. However, even for the amorphous solid, if the moisture content is controlled within certain range, the solid will possess better stability compared with the solid with other moisture content. pH is another key parameter for obtaining crystals with improved stability from the compound of formula I, and beyond the specified pH range, the substance will convert to amorphous form.

### Definition

As used herein, the term "effective amount" refers to a carrier for administration of a therapeutic agent, including various excipients and diluents. The term refers to such carriers that they themselves are not necessary active ingredients, and won't produce undue toxicity upon administration. Suitable carriers are well-known to the skilled person in the art. In "Remington's Pharmaceutical Sciences" (Mack Pub. Co., NJ 1991), a full discussion on pharmaceutically acceptable excipients can be found. In the composition, pharmaceutically acceptable carriers can include liquids such as water, saline, glycerol and ethanol. Additionally, auxiliary substances may be present with these carriers, such as disintegrating agents, wetting agents, emulsifying agents, pH buffering substances and the like.

The pharmaceutical composition can be prepared into a variety of dosage forms depending on the route of administration. The dosage forms are administered in the following manner: oral, inhalation spray, rectal, nasal, buccal, topical, parenteral, such as subcutaneous, intravenous, intramuscular, intraperitoneal, intrathecal, intraventricular, intrasternal and intracranial injection or infusion, or by means of reservoir explants.

As used herein, "compound of formula I" or "formula I compound" may be used interchangeably, both of which refer to a compound having the following structure formula or a pharmaceutically acceptable salt thereof: 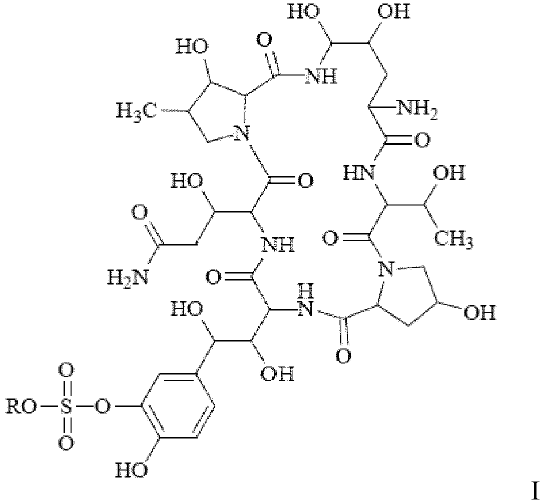 wherein, R represents H or a cation capable of forming a pharmaceutically acceptable salt.

Preferably, pharmaceutically acceptable salts include: metal salts such as alkali metal salts (such as sodium salt, potassium salt), alkaline earth metal salts (such as calcium salt, magnesium salt, etc.), ammonium salts, salts formed with organic bases (e.g., trimethylamine salt, triethylamine salt, pyridine salt, picoline salt, dicyclohexylamine salt, N,N,-dibenzylethylenediamine salt, diisopropylethylamine salt, etc.), organic acid addition salts (such as formate, acetate, trifluoroacetate, maleate, tartrate, methanesulfonate, benzenesulfonate, toluenesulfonate, etc.), inorganic acid addition salts (e.g. hydrochloride , hydrobromide, hydroiodide, sulfate, phosphate, etc.), salts formed with an amino acid (e.g. arginine, aspartic acid, glutamic acid, etc.), and the like. Preferably, R is H, a sodium ion or a diisopropylethylamine ion.

The compound of formula I can be obtained by conventional methods in the art, for example, but not limited to, the preparation method for this compound reported in WO9611210; alternatively, the compound may also be obtained through commercial sources, such as, but not limited to, such as Fujisawa, Japan.

As used herein, "C1-C4 lower alcohol" refers to alcohols, the number of carbon atoms of which is 1-4.

All the features mentioned above or in the examples below of the description can be optionally combined. All features disclosed in this specification may be used in any combination. Any alternative feature serving the same, equivalent, or similar purpose may replace each feature disclosed in this specification. Therefore, unless otherwise specified, the features as disclosed are only general examples of equivalent or similar features.

### Preparation of hydrates of the compound of formula I

After extensive researches, the inventors discovered that stable hydrates of the compound of formula I can be obtained by dissolving the compound into water or mixture solution of water-miscible organic solvents, maintaining the solubility of the solution comprising the compound of formula I around saturated, controlling pH value of the solution within specified range, and changing some factors, such as crystallization temperature, molar concentration, cooling rate or stirring rate, and crystallization time, and then vacuum-drying.

Based on the above findings, the present invention is completed by the inventors.

In the present invention, a stable hydrate of the compound of formula I is provided, wherein the mass percent of water in the hydrate 8.0%-30%; the most preferably 9.5%-28%.

In the present invention, a preparation method for a hydrate of the compound of formula I is provided, including the following steps:
(a) dissolving the compound of formula I into water or aqueous organic solvent (i), and controlling pH of the solution;
(b) obtaining the hydrate of the compound of formula I by reducing the temperature and / or adding organic solvent (i);
(c) obtaining the hydrate by centrifuging or filtrating;
(d) vacuum-drying the solid obtained in step (c) and controlling the mass percent of water in the hydrate.

Wherein,
In step (a), the temperature for dissolution is 10 to 50°C, preferably 20 to 40°C.

In step (a), the volume ratio of organic solvent (i) to water in the aqueous organic solvent (i) is 0.01 to 100, preferably 0.1 to 10, more preferably 0.5 to 3.0.

In step (a), the solution comprises 10 to 500 mg/ml, preferably 100 to 400 mg/ml of compound of formula I, based on the total volume of the solution in step (a).

In step (a), pH of the solution is controlled at 2.0-5.0, preferably 3.5-4.5.

In step (b), the temperature is reduced to -40 to 35°C, preferably -10 to 35°C, more preferably -5 to 30°C, and the most preferably 5 to 10°C.

In step (b), the volume ratio of organic solvent (i) to the solution of step (a) is 0.1 to 10, and preferably 1-5.

In step (a) and / or (b), said organic solvent (i) is C1-C4 lower alcohol; preferably, one or more selected from the group consisting of methanol, ethanol, n-propanol, and isopropanol.

In step (d), the mass percent of water in the solid is controlled at higher than 8.0%; preferably, within 8.0%-30%; the most preferably, within 9.5%-28%.

Upon complete crystallization, the hydrates can be separated by filtration, decanting solvent and the like, preferably, by filtration. The hydrates can be washed by water, and finally vacuum-dried, so as to obtain the hydrates of compound of formula I.

In one embodiment of the present invention, the hydrate of the compound of formula I is prepared through the following steps:
(a) dissolving the compound of formula I into water, and controlling pH of the solution;
(b) obtaining the hydrate of the compound of formula I by reducing the temperature;
(c) obtaining the hydrate by centrifuging or filtrating;
(d) vacuum-drying the solid obtained in step (c) and controlling the mass percent of water in the hydrate;
wherein,
in step (a), the temperature for dissolution is 10 to 50°C, preferably 20 to 40°C.

In step (a), the solution comprises 10 to 500 mg/ml, preferably 100 to 400 mg/ml of compound of formula I, based on the total volume of the solution in step (a).

In step (a), pH of the solution is controlled at 2.0-5.0, preferably 3.5-4.5.

In step (b), the temperature is reduced to -40 to 35°C, preferably -10 to 35°C, more preferably -5 to 30°C, and the most preferably 5 to 10°C.

In step (d), the mass percent of water in the solid is controlled at within 8.0%-30%; the most preferably, within 9.5%-28%.

In another embodiment of the present invention, the hydrate of the compound of formula I is prepared through the following steps:
(a) dissolving the compound of formula I into water, and controlling pH of the solution;
(b) obtaining the hydrate of the compound of formula I by adding organic solvent (i);
(c) obtaining the hydrate by centrifuging or filtrating;
(d) vacuum-drying the solid obtained in step (c) and controlling the mass percent of water in the hydrate;
wherein,
in step (a), the temperature for dissolution is 10 to 50°C, preferably 20 to 40°C.

In step (a), the solution comprises 10 to 500 mg/ml, preferably 50 to 300 mg/ml of compound of formula I, based on the total volume of the solution in step (a).

In step (a), pH of the solution is controlled at 2.0-5.0, preferably 3.5-4.5.

In step (b), said organic solvent (i) is C1-C4 lower alcohol; preferably, one or more selected from the group consisting of methanol, ethanol, n-propanol, and isopropanol.

In step (b), the volume ratio of organic solvent (i) to the solution of step (a) is 0.1 to 10, and preferably 1-5.

In step (d), the mass percent of water in the solid is controlled at within 8.0%-30%; the most preferably, within 9.5%-28%.

In another embodiment of the present invention, the hydrate of the compound of formula I is prepared through the following steps:
(a) dissolving the compound of formula I into water, and controlling pH of the solution;
(b) obtaining the hydrate of the compound of formula I by reducing the temperature and adding organic solvent (i);
(c) obtaining the hydrate by centrifuging or filtrating;
(d) vacuum-drying the solid obtained in step (c) and controlling the mass percent of water in the hydrate;
wherein,
in step (a), the temperature for dissolution is 10 to 50°C, preferably 20 to 40°C.

In step (a), the solution comprises 10 to 500 mg/ml, preferably 50 to 300 mg/ml of compound of formula I, based on the total volume of the solution in step (a).

In step (a), pH of the solution is controlled at 2.0-5.0, preferably 3.5-4.5.

In step (b), said organic solvent (i) is C1-C4 lower alcohol; preferably, one or more selected from the group consisting of methanol, ethanol, n-propanol, and isopropanol.

In step (b), the temperature is reduced to -40 to 35°C, preferably -10 to 35°C, more preferably -5 to 30°C, and the most preferably 5 to 10°C.

In step (b), the volume ratio of organic solvent (i) to the solution of step (a) is 0.1 to 10, and preferably 1-5.

In step (d), the mass percent of water in the solid is controlled within 8.0%-30%; the most preferably, within 9.5%-28%.

In another embodiment of the present invention, the hydrate of the compound of formula I is prepared through the following steps:
(a) dissolving the compound of formula I into aqueous organic solvent (i), and controlling pH of the solution;
(b) obtaining the hydrate of the compound of formula I by reducing the temperature;
(c) obtaining the hydrate by centrifuging or filtrating;
(d) vacuum-drying the solid obtained in step (c) and controlling the mass percent of water in the hydrate;
wherein,
in step (a), the temperature for dissolution is 10 to 50°C, preferably 20 to 40°C.

In step (a), the volume ratio of organic solvent (i) to water in the aqueous organic solvent (i) is 0.01 to 100, preferably 0.1 to 10, more preferably 0.5 to 3.0

In step (a), the solution comprises 10 to 500 mg/ml, preferably 100 to 400 mg/ml of compound of formula I, based on the total volume of the solution in step (a).

In step (a), pH of the solution is controlled at 2.0-5.0, preferably 3.5-4.5.

In step (a), said organic solvent (i) is C1-C4 lower alcohol; preferably, one or more selected from the group consisting of methanol, ethanol, n-propanol, and isopropanol.

In step (b), the temperature is reduced to -40 to 35°C, preferably -10 to 35°C, more preferably -5 to 30°C, and the most preferably 5 to 10°C.

In step (d), the mass percent of water in the solid is controlled at within 8.0%-30%; the most preferably, within 9.5%-28%.

In another embodiment of the present invention, the hydrate of the compound of formula I is prepared through the following steps:
(a) dissolving the compound of formula I into aqueous organic solvent (i), and controlling pH of the solution;
(b) obtaining the hydrate of the compound of formula I by adding organic solvent (i);
(c) obtaining the hydrate by centrifuging or filtrating;
(d) vacuum-drying the solid obtained in step (c) and controlling the mass percent of water in the hydrate;
wherein,
in step (a), the temperature for dissolution is 10 to 50°C, preferably 20 to 40°C.

In step (a), the volume ratio of organic solvent (i) to water in the aqueous organic solvent (i) is 0.01 to 100, preferably 0.1 to 10, more preferably 0.5 to 3.0.

In step (a), the solution comprises 10 to 500 mg/ml, preferably 100 to 400 mg/ml of compound of formula I, based on the total volume of the solution in step (a).

In step (a), pH of the solution is controlled at 2.0-5.0, preferably 3.5-4.5.

In step (b), the volume ratio of organic solvent (i) to the solution of step (a) is 0.1 to 10, and preferably 1-5.

In step (d), the mass percent of water in the solid is controlled at within 8.0%-30%; the most preferably, within 9.5%-28%.

In step (a) and (b), said organic solvent (i) is C1-C4 lower alcohol; preferably, one or more selected from the group consisting of methanol, ethanol, n-propanol, and isopropanol.

In step (d), the mass percent of water in the solid is controlled at within 8.0%-30%; the most preferably, within 9.5%-28%.

In another embodiment of the present invention, the hydrate of the compound of formula I is prepared through the following steps:
(a) dissolving the compound of formula I into aqueous organic solvent (i), and controlling pH of the solution;
(b) obtaining the hydrate of the compound of formula I by reducing the temperature and adding organic solvent (i);
(c) obtaining the hydrate by centrifuging or filtrating;
(d) vacuum-drying the solid obtained in step (c) and controlling the mass percent of water in the hydrate;
wherein,
in step (a), the temperature for dissolution is 10 to 50°C, preferably 20 to 40°C.

In step (a), the volume ratio of organic solvent (i) to water in the aqueous organic solvent (i) is 0.01 to 100, preferably 0.1 to 10, more preferably 0.5 to 3.0.

In step (a), the solution comprises 10 to 500 mg/ml, preferably 100 to 400 mg/ml of compound of formula I, based on the total volume of the solution in step (a).

In step (a), pH of the solution is controlled at 2.0-5.0, preferably 3.5-4.5.

In step (b), the temperature is reduced to -40 to 35°C, preferably -10 to 35°C, more preferably -5 to 30°C, and the most preferably 5 to 10°C.

In step (b), the volume ratio of organic solvent (i) to the solution of step (a) is 0.1 to 10, and preferably 1-5.

In step (a) and (b), said organic solvent (i) is C1-C4 lower alcohol; preferably, one or more selected from the group consisting of methanol, ethanol, n-propanol, and isopropanol.

In step (d), the mass percent of water in the solid is controlled at within 8.0%-30%; the most preferably, within 9.5%-28%.

### Identification and properties

After the hydrate of compound of formula I was obtained, the properties thereof were studied by the inventors using various methods and instruments.

In one embodiment of the invention, general detecting methods in the art are used to detect the mass percent of water in a composition. For example, Karl Fischer (KF) determination is used to detect moisture content.

In another embodiment of the invention, by using HPLC, the purity of the sample prepared by the method of the invention was detected, and the stability of the sample was studied. The HPLC method is listed as follows:
HPLC: Waters 1525-717-2498
Chromatographic Column: ACE 3 AQ, 150 × 4.6mm, 3 µm
Mobile phase: A: 1000 ml of water, 10 ml of methanol, 100 µl of trifluoroacetic acid
B: 600 ml of water, 400ml of methanol, 100 µl of trifluoroacetic acid (the used reagents are HPLC grade, supplied by TEDIA company, inc.)
Flow rate: 0.55 ml/min
Column temperature: 50°C
Gradient:

| time | Mobile phase A | Mobile phase B |
|---|---|---|
| min | % | % |
| 0 | 100 | 0 |
| 25 | 100 | 0 |
| 55 | 55 | 45 |
| 56 | 0 | 100 |
| 61 | 0 | 100 |
| 62 | 100 | 0 |
| 70 | 100 | 0 |

Injector temperature: 5°C
Detection wavelength: 225 nm

### Study on the stability of the hydrate of compound of formula I

The hydrate of compound of formula I according to the invention is stable, suitable for industrial production and favorable to transportation and preservation. Through stability test, the hydrate of compound of formula I prepared by the method according to the invention was determined as having good stability by the inventors. The hydrate can be stored at 25°C for long-term, and solve the transportation problem for APIs.

Upon intensive research, the inventors further discovered that the stability of the hydrate of compound of formula I drug stability is closely related to the moisture content. When the moisture content is higher than 8.0%, the hydrate will possess good stability, and can be stored at 25°C for a long term.

When the moisture content is less than 8.0%, the product can be stored at 0-8°C for a long term with slight decomposition. However, if the product is stored at 25°C for a long term, there will be significant decomposition.

### Uses

Uses of the hydrate of compound of formula I are provided in the invention. In one aspect, it can be used to prepare the compound of formula II. The synthesis processes have been reported in literatures such as WO9611210 WO03018615 and WO2004014879.

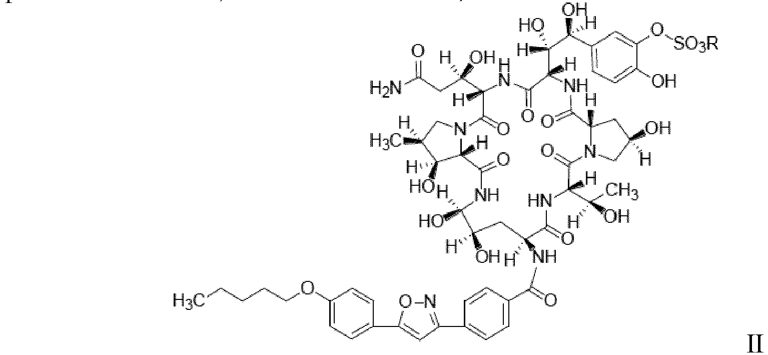

Based on the above results, a pharmaceutical composition is further provided in the present invention, comprising the hydrate of compound of formula I and a pharmaceutically acceptable carrier.

### The advantages of the invention mainly include:

1. The inventors have selected particular preparation conditions through repeated experiments, and unexpected technical effects have produced, so that a preparation method for the high-stability hydrate of compound of formula I is provided, and such method is suitable for large-scale production.
2. The hydrate of compound of formula I possesses excellent stability, and is significantly superior to the compound of formula I, the mass percent of water of which is less than 8.0%, and to the compound of formula I prepared in prior art.
3. The operation of the process of the invention is simple, and the obtained high-stability hydrate is favorable to transportation and preservation, so as to reduce the production cost and produce unexpected technical effects.

The invention will be further illustrated with reference to the following specific examples. It is to be understood that these examples are only intended to illustrate the invention. For the experimental methods in the following examples without particular conditions, they are performed under routine conditions or as instructed by the manufacturer. Unless otherwise specified, all percentages, ratios, proportions or parts are by weight.

The unit of the weight/volume percentages in the invention is well known to the skilled in the art, for example, the weight of a solute in a 100 mL solution.

Unless otherwise defined, all scientific and technical terms used herein have the same meaning as commonly understood by the skilled in the art. Furthermore, any process or material similar or equivalent to those described herein can be used in the process of the present invention. The preferred embodiments and materials described herein are merely provided for illustration.

### Example 1

### Preparation of compound I

153 g of the compound of formula I in solid powder was prepared according to the method of Example 1 in U.S. Patent No. 5,376,634. Moisture content of the compound of formula I was determined as 3.4% by Karl Fischer method. 2.0 g of the above obtained sample was taken for stability study. The study is conducted as follows: the sample is place in a sealed container at 0-8°C for 6 months and at 25°C for 6 months, respectively; and then the amount of impurities is analyzed. Initially, the amount of impurities in the compound of formula I is 2.4%; after placed at 0-8°C for 6 months, the amount of impurities in the sample is 3.0%; and after placed at 25°C for 6 months, the amount of impurities in the sample is 4.9%.

### Example 2

### Preparation of hydrates A, B, C, D, and E comprising the compound of formula I

At 50°C, 7.0 g of solid powder of compound of formula I prepared in Example 1 was dissolved into a mixed solution consisting of 10 ml of water and 8 ml of n-propanol, and stirred for 30 mins to completely dissolve the compound of formula I. pH was adjusted to 3.5 by using glacial acetic acid. The solution was cooled to 25°C, and hydrates of the compound of formula I precipitated. The system was stirred for 5 hours at 25°C, so that the hydrate of compound of formula I gradually grew. And then 36 ml of n-propanol was slowly added dropwise, and the resulting system was stirred at 25°C for 2 hours. The hydrate of compound of formula I was obtained by filtration. The hydrate of compound of formula I was vacuum-dried at 20°C-25°C for 1 hour. 1.0 g of hydrate was taken and named as hydrate A of compound of formula I, and the mass percent of water in hydrate A was determined as 29.5%. The remaining sample was further dried for 0.5 hour. 1.0 g of hydrate was taken and named as hydrate B of compound of formula I, and the mass percent of water in hydrate B was determined as 27.1%. The remaining sample was further dried for 3 hours. 1.0 g of hydrate was taken and named as hydrate C of compound of formula I, and the mass percent of water in hydrate C was determined as 12.5%. The remaining sample was further dried for 1.5 hours. 1.0 g of hydrate was taken and named as hydrate D of compound of formula I, and the mass percent of water in hydrate D was determined as 9.5%. P₂O₅ was placed in vacuum oven, and the remaining sample was further dried for 2 hours. 1.0 g of hydrate was taken and named as hydrate E of compound of formula I, and the mass percent of water in hydrate D was determined as 6.1%.

Stability study was applied to the above obtained samples as follows: hydrate A, hydrate B, hydrate C, hydrate D, hydrate E were place in sealed containers at 0-8°C for 6 months, and at 25°C for 6 months, respectively; and then the amounts of impurities are analyzed.

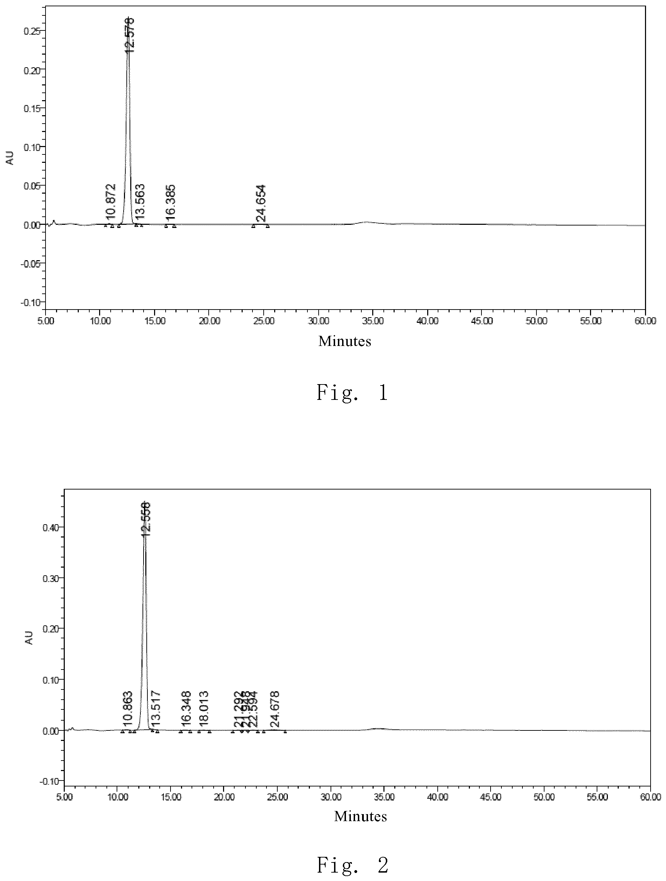

The data in HPLC pattern (fig. 1) for hydrate D after placed at 25°C for 6 months are shown in the following table:

| | Name | Retention Time (Min) | Relative Retention Time | Peak Area% |
|---|---|---|---|---|
| 1 | Impurity | 10.87 | 0.87 | 0.17 |
| 2 | Compound I | 12.58 | 1.00 | 99.60 |
| 3 | Impurity | 13.56 | 1.08 | 0.08 |
| 4 | Impurity | 16.39 | 1.30 | 0.07 |
| 5 | Impurity | 24.65 | 1.97 | 0.08 |

Stability results are shown in the following table:

| Sample | Moisture content in sample | Initial impurity content in sample | Experiment Conditions | |
|---|---|---|---|---|
| | | | Impurity content in sample, 0-8°C, 6 months | Impurity content in sample, 25°C, 6 months (Fig. 3) |
| Hydrate A | 29.5% | 0.4% | 0.4% | 0.6% |
| Hydrate B | 27.1% | 0.4% | 0.4% | 0.5% |
| Hydrate C | 12.5% | 0.4% | 0.4% | 0.4% |
| Hydrate D | 9.5% | 0.4% | 0.4% | 0.4% |
| Hydrate E | 6.1% | 0.5% | 0.9% | 1.9% |

### Example 3

### Preparation of hydrates F, G, H, I, and J comprising the compound of formula I

At 30°C, 16 g of compound of formula I prepared in Example 1 was dissolved into 90 ml of water, and stirred for 2 hours to completely dissolve the compound of formula I. pH was adjusted to 2.0 by using glacial acetic acid. 610 ml of ethanol was slowly added dropwise, and hydrates of the compound of formula I precipitated. The solution was cooled to 11°C, and stirred at 11°C for 2 hours. The hydrate of compound of formula I was obtained by filtration. The hydrate of compound of formula I was vacuum-dried at 20°C-25°C for 0.5 hour. 1.0 g of hydrate was taken and named as hydrate F of compound of formula I, and the mass percent of water in hydrate F was determined as 31.2%. The remaining sample was further dried for 0.5 hour. 1.0 g of hydrate was taken and named as hydrate G of compound of formula I, and the mass percent of water in hydrate G was determined as 26.2%. The remaining sample was further dried for 2 hours. 1.0 g of hydrate was taken and named as hydrate H of compound of formula I, and the mass percent of water in hydrate H was determined as 14.6%. The remaining sample was further dried for 2 hours. 1.0 g of hydrate was taken and named as hydrate I of compound of formula I, and the mass percent of water in hydrate I was determined as 8.6%. P₂O₅ was placed in vacuum oven, and the remaining sample was further dried for 1 hour. 1.0 g of hydrate was taken and named as hydrate J of compound of formula I, and the mass percent of water in hydrate J was determined as 7.2%.

Stability results are shown in the following table:

| Sample | Moisture content in sample | Initial impurity content in sample | Experiment Conditions | |
|---|---|---|---|---|
| | | | Impurity content in sample, 0-8 °C, 6 months | Impurity content in sample, 25°C, 6 months (Fig. 4) |
| Hydrate F | 31.2% | 0.4% | 0.7% | 1.2% |
| Hydrate G | 26.2% | 0.4% | 0.4% | 0.4% |
| Hydrate H | 14.6% | 0.4% | 0.4% | 0.5% |
| Hydrate I | 8.6% | 0.4% | 0.5% | 0.6% |
| Hydrate J | 7.2% | 0.5% | 0.8% | 2.1% |

### Example 4

### Preparation of hydrates K, L, M, N, and O comprising the compound of formula I

At 28°C, 18 g of compound of formula I prepared in Example 1 was dissolved into a mixed solution consisting of 50 ml of water and 50 ml of isopropanol, and stirred for 1 hour to completely dissolve the compound of formula I. pH was adjusted to 3.6 by using glacial acetic acid. The solution was cooled to 17°C, and hydrates of the compound of formula I precipitated. The system was further cooled to -10°C and stirred for more than 2 hours. The hydrate of compound of formula I was obtained by filtration. The hydrate of compound of formula I was vacuum-dried at 20°C-25°C for 0.5 hour. 1.0 g of hydrate was taken and named as hydrate K of compound of formula I, and the mass percent of water in hydrate K was determined as 29.5%. The remaining sample was further dried for 0.5 hour. 1.0 g of hydrate was taken and named as hydrate L of compound of formula I, and the mass percent of water in hydrate L was determined as 27.5%. The remaining sample was further dried for 3 hours. 1.0 g of hydrate was taken and named as hydrate M of compound of formula I, and the mass percent of water in hydrate M was determined as 19.8%. The remaining sample was further dried for 4 hours. 1.0 g of hydrate was taken and named as hydrate N of compound of formula I, and the mass percent of water in hydrate N was determined as 9.6%. P₂O₅ was placed in vacuum oven, and the remaining sample was further dried for 4 hours. 1.0 g of hydrate was taken and named as hydrate O of compound of formula I, and the mass percent of water in hydrate O was determined as 4.9%.

Stability results are shown in the following table:

| Sample | Moisture content in sample | Initial impurity content in sample | Experiment Conditions | |
|---|---|---|---|---|
| | | | Impurity content in sample, 0-8 °C, 6 months | Impurity content in sample, 25°C, 6 months (Fig. 5) |
| Hydrate K | 29.5% | 0.4% | 0.4% | 0.6% |
| Hydrate L | 27.5% | 0.4% | 0.4% | 0.4% |
| Hydrate M | 19.8% | 0.4% | 0.4% | 0.4% |
| Hydrate N | 9.6% | 0.4% | 0.5% | 0.5% |
| Hydrate O | 4.9% | 0.6% | 0.9% | 2.4% |

### Example 5

### Preparation of hydrates P, Q, R, S, and T comprising the compound of formula I

At 25°C, 10.0 g of compound of formula I prepared in Example 1 was dissolved into a mixed solution consisting of 40 ml of water and 64 ml of methanol, and stirred for 2 hours to completely dissolve the compound of formula I. pH was adjusted to 3.5 by using glacial acetic acid. 300 ml of methanol was slowly added dropwise, and hydrates of the compound of formula I precipitated. The hydrate of compound of formula I was obtained by filtration. The hydrate of compound of formula I was vacuum-dried at 20°C-25°C (stirred for 2 hours) for 0.5 hour. 1.0 g of hydrate was taken and named as hydrate P of compound of formula I, and the mass percent of water in hydrate P was determined as 31.3%. The remaining sample was further dried for 0.5 hour. 1.0 g of hydrate was taken and named as hydrate Q of compound of formula I, and the mass percent of water in hydrate Q was determined as 27.3%. The remaining sample was further dried for 3 hours. 1.0 g of hydrate was taken and named as hydrate R of compound of formula I, and the mass percent of water in hydrate R was determined as 19.0%. The remaining sample was further dried for 4 hours. 1.0 g of hydrate was taken and named as hydrate S of compound of formula I, and the mass percent of water in hydrate S was determined as 9.0%. P₂O₅ was placed in vacuum oven, and the remaining sample was further dried for 1 hour. 1.0 g of hydrate was taken and named as hydrate T of compound of formula I, and the mass percent of water in hydrate T was determined as 8%.

Stability results are shown in the following table:

| Sample | Moisture content in sample | Initial impurity content in sample | Experiment Conditions | |
|---|---|---|---|---|
| | | | Impurity content in sample, 0-8 °C, 6 months | Impurity content in sample, 25°C, 6 months (Fig. 6) |
| Hydrate P | 31.3% | 0.3% | 0.4% | 0.9% |
| Hydrate Q | 27.3% | 0.3% | 0.3% | 0.4% |
| Hydrate R | 19.0% | 0.3% | 0.3% | 0.3% |
| Hydrate S | 9.0% | 0.4% | 0.4% | 0.5% |
| Hydrate T | 8.0% | 0.4% | 0.5% | 0.6% |

### Example 6

### Preparation of hydrates U, V, W, X, and Y comprising the compound of formula I

At 40°C, 15 g of compound of formula I prepared in Example 1 was dissolved into 50 ml of water, and stirred to completely dissolve the compound of formula I. pH was adjusted to 4.0 by using glacial acetic acid. The solution was cooled to 22°C, and hydrates of the compound of formula I precipitated. The system was further cooled to 5°C and stirred for 10 hours at 5°C. The hydrate of compound of formula I was obtained by filtration. The hydrate of compound of formula I was vacuum-dried at 20°C-25°C for 1 hour. 1.0 g of hydrate was taken and named as hydrate U of compound of formula I, and the mass percent of water in hydrate U was determined as 42.0%. The remaining sample was further dried for 2 hours. 1.0 g of hydrate was taken and named as hydrate V of compound of formula I, and the mass percent of water in hydrate V was determined as 30.0%. The remaining sample was further dried for 2 hours. 1.0 g of hydrate was taken and named as hydrate W of compound of formula I, and the mass percent of water in hydrate W was determined as 19.5%. P₂O₅ was placed in vacuum oven, and the remaining sample was further dried for 2 hours. 1.0 g of hydrate was taken and named as hydrate X of compound of formula I, and the mass percent of water in hydrate X was determined as 9.6%. P₂O₅ was placed in vacuum oven, and the remaining sample was further dried for 2 hours. 1.0 g of hydrate was taken and named as hydrate Y of compound of formula I, and the mass percent of water in hydrate Y was determined as 1.9%.

Stability results are shown in the following table:

| Sample | Moisture content in sample | Initial impurity content in sample | Experiment Conditions | |
|---|---|---|---|---|
| | | | Impurity content in sample, 0-8 °C, 6 months | Impurity content in sample, 25°C, 6 months (Fig. 7) |
| Hydrate U | 42.0% | 0.3% | 0.7% | 1.5% |
| Hydrate V | 30.0% | 0.3% | 0.3% | 0.3% |
| Hydrate W | 19.5% | 0.3% | 0.3% | 0.3% |
| Hydrate X | 9.6% | 0.3% | 0.3% | 0.3% |
| Hydrate Y | 1.9% | 0.4% | 1.0% | 2.3% |

The data in HPLC pattern (fig. 2) for hydrate Y after placed at 25°C for 6 months are shown in the following table:

| | Name | Retention Time (Min) | Relative Retention Time | Peak Area% |
|---|---|---|---|---|
| 1 | Impurity | 10.86 | 0.87 | 0.17 |
| 2 | Compoun d I | 12.56 | 1.00 | 97.69 |
| 3 | Impurity | 13.52 | 1.08 | 0.08 |
| 4 | Impurity | 16.35 | 1.30 | 0.07 |
| 5 | Impurity | 18.01 | 1.43 | 0.02 |
| 6 | Impurity | 21.29 | 1.69 | 0.04 |
| 7 | Impurity | 21.95 | 1.74 | 0.13 |
| 8 | Impurity | 22.59 | 1.80 | 0.15 |
| 9 | Impurity | 24.68 | 1.97 | 1.65 |

### Example 7

### Preparation of hydrates a, b, and c comprising the compound of formula I

At 40°C, 15 g of compound of formula I prepared in Example 1 was dissolved into 50 ml of water, and stirred to completely dissolve the compound of formula I. pH was adjusted to 5.0 by using glacial acetic acid. The solution was cooled to 22°C, and the hydrate of compound of formula I precipitated. 150 ml of ethanol was slowly added, and stirred for 2 hours. The hydrate of compound of formula I was obtained by filtration. The hydrate of compound of formula I was vacuum-dried at 20°C-25°C for 1 hour. 1.0 g of hydrate was taken and named as hydrate a of compound of formula I, and the mass percent of water in hydrate a was determined as 42.0%. The remaining sample was further dried for 3.5 hours. 1.0 g of hydrate was taken and named as hydrate b of compound of formula I, and the mass percent of water in hydrate b was determined as 17.5%. P₂O₅ was placed in vacuum oven, and the remaining sample was further dried for 3 hours. 1.0 g of hydrate was taken and named as hydrate c of compound of formula I, and the mass percent of water in hydrate c was determined as 6.3%.

Stability results are shown in the following table:

| Sample | Moisture content in sample | Initial impurity content in sample | Experiment Conditions | |
|---|---|---|---|---|
| | | | Impurity content in sample, 0-8 °C , 6 months | Impurity content in sample, 25°C, 6 months |
| Hydrate a | 42.0% | 0.3% | 0.6% | 1.0% |
| Hydrate b | 17.5% | 0.3% | 0.3% | 0.3% |
| Hydrate c | 6.3% | 0.3% | 0.6% | 1.3% |

### Example 8

### Preparation of hydrates d, e, and f comprising the compound of formula I

At 20°C, 12 g of compound of formula I prepared in Example 1 was dissolved into 40 ml of water, and stirred to completely dissolve the compound of formula I. pH was adjusted to 4.5 by using glacial acetic acid. 180 ml of n-propanol was slowly added and stirred for 2 hours, and hydrates of the compound of formula I precipitated. The hydrate of compound of formula I was obtained by filtration. The system was stirred for more than 2 hours. The hydrate of compound of formula I was obtained by filtration. The hydrate of compound of formula I was vacuum-dried at 20°C-25°C for 1 hour. 1.0 g of hydrate was taken and named as hydrate d of compound of formula I, and the mass percent of water in hydrate d was determined as 31.0%. The remaining sample was further dried for 4 hours. 1.0 g of hydrate was taken and named as hydrate e of compound of formula I, and the mass percent of water in hydrate e was determined as 9.2%. P₂O₅ was placed in vacuum oven, and the remaining sample was further dried for 4 hours. 1.0 g of hydrate was taken and named as hydrate f of compound of formula I, and the mass percent of water in hydrate f was determined as 1.3%.

Stability results are shown in the following table:

| Sample | Moisture content in sample | Initial impurity content in sample | Experiment Conditions | |
|---|---|---|---|---|
| | | | Impurity content in sample, 0-8 °C, 6 months | Impurity content in sample, 25°C, 6 months |
| Hydrate d | 31.0% | 0.3% | 0.5% | 0.7% |
| Hydrate e | 9.2% | 0.3% | 0.3% | 0.3% |
| Hydrate f | 1.3% | 0.3% | 0.7% | 2.2% |

It can be concluded from the above examples that the hydrates of compound of formula I, the moisture content of which are 8.0% -30%, will possess excellent stability. If the moisture content of the hydrate of compound of formula I is higher than 30% or less than 8.0%, the stability thereof will significantly decreased.

### Example 9

### Preparation of hydrates g, h, and i comprising the compound of formula I (effects of pH)

At 30°C, 12 g of compound I prepared in Example 1 was dissolved into 60 ml of water, and stirred to dissolve the compound. pH was adjusted to 1.8 by using glacial acetic acid. 200 ml of ethanol was slowly added, and solids of the compound I precipitated. The system was stirred for another 1 hour, and filtrated. The hydrate of compound of formula I was vacuum-dried at 20°C-25°C for 1 hour. 1.0 g of hydrate was taken and named as hydrate g of compound of formula I, and the mass percent of water in hydrate g was determined as 36.0%. The remaining sample was further dried for 4 hours. 1.0 g of hydrate was taken and named as hydrate h of compound of formula I, and the mass percent of water in hydrate h was determined as 14.5%. P₂O₅ was placed in vacuum oven, and the remaining sample was further dried for 4 hours. 1.0 g of hydrate was taken and named as hydrate I of compound of formula I, and the mass percent of water in hydrate i was determined as 6.3%.

Stability results are shown in the following table:

| Sample | Moisture content in sample | Initial impurity content in sample | Experiment Conditions | |
|---|---|---|---|---|
| | | | Impurity content in sample, 0-8 °C, 6 months | Impurity content in sample, 25°C, 6 months |
| Hydrate g | 36.0% | 1.7% | 2.9% | 3.9% |
| Hydrate h | 14.5% | 1.7% | 1.9% | 2.6% |
| Hydrate i | 6.3% | 1.8% | 2.9% | 4.1% |

### Example 10

### Preparation of hydrates j, k, and 1 comprising the compound of formula I (effects of pH)

At 30°C, 12 g of compound I prepared in Example 1 was dissolved into 60 ml of water, and stirred to dissolve the compound. pH was adjusted to 5.4 by using glacial acetic acid. 200 ml of ethanol was slowly added, and solids of the compound I precipitated. The system was stirred for another 1 hour, and filtrated. The hydrate of compound of formula I was vacuum-dried at 20°C-25°C for 1 hour. 1.0 g of hydrate was taken and named as hydrate j of compound of formula I, and the mass percent of water in hydrate j was determined as 35.0%. The remaining sample was further dried for 4 hours. 1.0 g of hydrate was taken and named as hydrate k of compound of formula I, and the mass percent of water in hydrate k was determined as 14.1%. P₂O₅ was placed in vacuum oven, and the remaining sample was further dried for 4 hours. 1.0 g of hydrate was taken and named as hydrate 1 of compound of formula I, and the mass percent of water in hydrate 1 was determined as 6.6%.

Stability results are shown in the following table:

| Sample | Moisture content in sample | Initial impurity content in sample | Experiment Conditions | |
|---|---|---|---|---|
| | | | Impurity content in sample, 0-8 °C, 6 months | Impurity content in sample, 25°C, 6 months |
| Hydrate j | 35.0% | 1.9% | 3.1% | 5.6% |
| Hydrate k | 14.1% | 2.0% | 2.4% | 3.0% |
| Hydrate 1 | 6.6% | 2.1% | 3.2% | 5.7% |

### Example 11

### Preparation of hydrates m, n, and o comprising the compound of formula I (effects of solvent)

At 20°C, 4.8 g of compound I prepared in Example 1 was dissolved into 14 ml of water. pH was adjusted to 4.0 by using glacial acetic acid. The resulting system was stirred for 2 hours to completely dissolve compound I. 35 ml of acetonitrile was slowly added and stirred for 2 hours, and solids precipitated. The system was stirred for another 2 hours, and filtrated. The hydrate of compound of formula I was vacuum-dried at 20°C-25°C for 1 hour. 1.0 g of hydrate was taken and named as hydrate m of compound of formula I, and the mass percent of water in hydrate m was determined as 25.0%. The remaining sample was further dried for 4 hours. 1.0 g of hydrate was taken and named as hydrate n of compound of formula I, and the mass percent of water in hydrate n was determined as 18.1%. P₂O₅ was placed in vacuum oven, and the remaining sample was further dried for 4 hours. 1.0 g of hydrate was taken and named as hydrate o of compound of formula I, and the mass percent of water in hydrate o was determined as 10.2%.

Stability results are shown in the following table:

| Sample | Moisture content in sample | Initial impurity content in sample | Experiment Conditions | |
|---|---|---|---|---|
| | | | Impurity content in sample, 0-8 °C, 6 months | Impurity content in sample, 25°C, 6 months |
| Hydrate m | 25.0% | 2.1% | 2.5% | 3.2% |
| Hydrate n | 18.1% | 2.2% | 2.5% | 3.3% |
| Hydrate o | 10.2% | 2.2% | 2.5% | 3.3% |

### Example 12

### Preparation of hydrates p, q, and r comprising the compound of formula I (effects of solvent)

At 18°C, 4.2 g of compound I prepared in Example 1 was dissolved into 14 ml of water. pH was adjusted to 4.0 by using glacial acetic acid. The resulting system was stirred for 1 hour to completely dissolve compound I. 40 ml of acetone was slowly added and stirred for 2 hours, and solids precipitated. The system was stirred for another 2 hours, and filtrated. The hydrate of compound of formula I was vacuum-dried at 20°C-25°C for 1 hour. 1.0 g of hydrate was taken and named as hydrate p of compound of formula I, and the mass percent of water in hydrate p was determined as 23.8%. The remaining sample was further dried for 4 hours. 1.0 g of hydrate was taken and named as hydrate q of compound of formula I, and the mass percent of water in hydrate q was determined as 15.6%. P₂O₅ was placed in vacuum oven, and the remaining sample was further dried for 4 hours. 1.0 g of hydrate was taken and named as hydrate r of compound of formula I, and the mass percent of water in hydrate r was determined as 7.6%.

Stability results are shown in the following table:

| Sample | Moisture content in sample | Initial impurity content in sample | Experiment Conditions | |
|---|---|---|---|---|
| | | | Impurity content in sample, 0-8 °C, 6 months | Impurity content in sample, 25°C, 6 months |
| Hydrate p | 23.8% | 2.1% | 3.1% | 5.2% |
| Hydrate q | 15.6% | 2.2% | 3.1% | 5.3% |
| Hydrate r | 7.6% | 2.2% | 4.2% | 7.6% |

It can be concluded from the above examples that pH and solvent will significantly affect the stability of hydrate. If pH is not controlled within 2.0-5.0, and the used solvent is not those used in the present invention, the stability of hydrate will significantly decreased. However, even for the above conditions, the hydrate of compound of formula I, the moisture content of which is within 8.0%-30%, will possess better stability, compared with the hydrate of compound of formula I, the moisture content of which is higher than 30% or less than 8.0%.

### Example 13

### Preparation of the compound of formula II

The compound of formula II was synthesized from the compound of formula I according to the process for synthesizing Micafungin in WO2004014879.

Hydrate A of the compound of formula I obtained in Example 2 of the present application (1.07 mmol, 1.00 g) was dissolved in 12 ml of DMF. The resulting solution was cooled to below 0°C in an ice bath. Diisopropylethylamine (0.22 g, 1.67 mmol) was added, and the temperature was kept at 0°C. MKC-8 (1-[4-[5-(4-pentyloxyphenyl)isoxazol-3-yl]benzoyloxy]-1H-1,2,3-benzotriazole) (0.53 g, 1.14 mmol) was slowly added, and the reaction was warmed to 2-6°C, and maintained for 4 hours. 60 ml of ethyl acetate was added directly into the reaction liquid at the end of the reaction, stirred for another 1 hour, and filtered, so as to give micafungin diisopropylethylamine. The salt was dissolved in 30 ml of acetone and 30 ml of ethyl acetate, starching and filtered. Micafungin diisopropylethylamine was dried in vacuo to remove residual organic solvent. The purity of Micafungin diisopropylethylamine was determined as 99.35% by HPLC, and the yield was 91.9%.

### Example 14

### Preparation of the compound of formula II from hydrates B, C, D, H, N, S of compound of formula I

The compound of formula II was synthesized from the compound of formula I according to the process for Micafungin synthesis in WO2004014879.

Hydrates B, C, D, H, N and S of compound of formula I obtained in Example 2, Example 3, Example 4, Example 5 of the present application (1.07 mmol, 1.00 g) were dissolved in 12 ml of DMF, respectively. The resulting solution was cooled to below 0 °C in an ice bath. Diisopropylethylamine (0.22 g, 1.67 mmol) was added respectively, and the temperature was kept at 0°C. MKC-8 (1-[4-[5-(4-pentyloxyphenyl)isoxazol-3-yl]benzoyloxy]-1H-1,2,3-benzotriazole) (0.53 g, 1.14 mmol) was slowly added, and the reaction was warmed to 2-6°C, and maintained for 4 hours. 60 ml of ethyl acetate was added directly into each reaction liquid at the end of the reaction, stirred for another 1 hour, and filtered, so as to give micafungin diisopropylethylamine. The salt obtained above was dissolved in 30 ml of acetone and 30 ml of ethyl acetate, starching and filtered. Micafungin diisopropylethylamine was dried in vacuo to remove residual organic solvent. The purity and yield of Micafungin diisopropylethylamine determined by HPLC are shown in the following table.

| Hydrate | B | C | D | H | N | S |
|---|---|---|---|---|---|---|
| HPLC purity% | 99.42% | 99.44% | 99.45% | 99.42% | 99.32% | 99.38% |
| Yield% | 93.2% | 95.1% | 94.5% | 98.0% | 93.9% | 96.5% |

### Comparative Example 1

### Preparation of the compound of formula II from the hydrate of compound of formula I with moisture content less than 8%

The compound of formula II was synthesized from the compound of formula I according to the process for Micafungin synthesis in WO2004014879.

Hydrates E, J, O, Y, c and f of compound of formula I obtained in Example 2, Example 3, Example 4, Example 6 and Example 8 of the present application (1.07 mmol, 1.00 g) were dissolved in 12 ml of DMF, respectively. The resulting solution was cooled to below 0°C in an ice bath. Diisopropylethylamine (0.22 g, 1.67 mmol) was added respectively, and the temperature was kept at 0°C. MKC-8 (1-[4-[5-(4-pentyloxyphenyl)isoxazol-3-yl]benzoyloxy]-1H-1,2,3-benzotriazole) (0.53 g, 1.14 mmol) was slowly added, and the reaction was warmed to 2-6°C, and maintained for 4 hours. 60 ml of ethyl acetate was added into each reaction liquid at the end of the reaction, stirred for another 1 hour, and filtered, so as to give micafungin diisopropylethylamine. The salt was dissolved in 30 ml of acetone and 30 ml of ethyl acetate, starching and filtered. Micafungin diisopropylethylamine was dried in vacuo to remove residual organic solvent. The purity and yield of Micafungin diisopropylethylamine determined bv HPLC are shown in the following table.

| Hydrate | E | J | O | Y | c | f |
|---|---|---|---|---|---|---|
| HPLC purity% | 99.10% | 99.04% | 99.00% | 98.82% | 99.12% | 98.78% |
| Yield% | 87.9% | 85.1% | 84.5% | 88.0% | 83.2% | 86.7% |

It can be concluded from the above comparative examples that the purity HPLC and yield of the compound of formula II decreased, when the hydrate of compound of formula I, the moisture content of which is less than 8%, was used.

### Comparative Example 2

### Preparation of the compound of formula II from the hydrate of compound of formula I with moisture content higher than 30%

The compound of formula II was synthesized from the compound of formula I according to the process for Micafungin synthesis in WO2004014879.

Hydrates F, P, U, a, d and g of compound of formula I obtained in Example 3, Example 5, Example 6, Example 7, Example 8 and Example 9 of the present application (1.07 mmol, 1.00 g) were dissolved in 12 ml of DMF, respectively. The resulting solution was cooled to below 0°C in an ice bath. Diisopropylethylamine (0.22 g, 1.67 mmol) was added respectively, and the temperature was kept at 0°C. MKC-8 (1-[4-[5-(4-pentyloxyphenyl)isoxazol-3-yl]benzoyloxy]-1H-1,2,3-benzotriazole) (0.53 g, 1.14 mmol) was slowly added, and the reaction was warmed to 2-6°C, and maintained for 4 hours. 60 ml of ethyl acetate was added directly into each reaction liquid at the end of the reaction, stirred for another 1 hour, and filtered, so as to give micafungin diisopropylethylamine. The salt was dissolved in 30 ml of acetone and 30 ml of ethyl acetate, starching and filtered. Micafungin diisopropylethylamine was dried in vacuo to remove residual organic solvent. The purity and yield of Micafungin diisopropylethylamine determined by HPLC are shown in the following table.

| Hydrate | F | P | U | a | d | g |
|---|---|---|---|---|---|---|
| HPLC purity% | 99.19% | 98.99% | 99.13% | 98.87% | 99.06% | 98.88% |

| | | | | | | |
|---|---|---|---|---|---|---|
| Yield% | 83.1% | 85.6% | 80.5% | 81.8% | 83.2% | 76.7% |

It can be concluded from the above comparative examples that the purity HPLC and yield of the compound of formula II decreased, when the hydrate of compound of formula I, the moisture content of which is higher than 30%, was used.

### Comparative Example 3

### Preparation of the compound of formula II from the hydrate of compound of formula I in Example 1

The compound of formula II was synthesized from the compound of formula I according to the process for Micafungin synthesis in WO2004014879.

Hydrate of compound of formula I obtained in Example 1 (1.07 mmol, 1.00 g) was dissolved in 12 ml of DMF, respectively. The resulting solution was cooled to below 0°C in an ice bath. Diisopropylethylamine (0.22 g, 1.67 mmol) was added respectively, and the temperature was kept at 0°C. MKC-8 (1-[4-[5-(4-pentyloxyphenyl)isoxazol-3-yl]benzoyloxy]-1H-1,2,3-benzotriazole) (0.53 g, 1.14 mmol) was slowly added, and the reaction was warmed to 2-6°C, and maintained for 4 hours. 60 ml of ethyl acetate was added directly into reaction liquid at the end of the reaction, stirred for another 1 hour, and filtered, so as to give micafungin diisopropylethylamine. The salt was dissolved in 30 ml of acetone and 30 ml of ethyl acetate, starching and filtered. Micafungin diisopropylethylamine was dried in vacuo to remove residual organic solvent. The purity of Micafungin diisopropylethylamine determined by HPLC was 95.7%, and the yield is 75.2%.

It can be concluded from the above comparative examples that the purity HPLC and yield of the compound of formula II significantly decreased, when the hydrate of compound of formula I in Example 1 was used.

### Example 15

### Preparation of the compound of formula II from the hydrate of compound of formula I

The compound of formula II was synthesized from the compound of formula I according to the process for Micafungin synthesis in WO2004014879.

Hydrates h, i, j, k, q and r of compound of formula I obtained in Example 9, Example 10 and Example 12 of the present application (1.07 mmol, 1.00 g) were dissolved in 12 ml of DMF, respectively. The resulting solution was cooled to below 0 °C in an ice bath. Diisopropylethylamine (0.22 g, 1.67 mmol) was added respectively, and the temperature was kept at 0°C. MKC-8 (1-[4-[5-(4-pentyloxyphenyl)isoxazol-3-yl]benzoyloxy]-1H-1,2,3-benzotriazole) (0.53 g, 1.14 mmol) was slowly added, and the reaction was warmed to 2-6°C, and maintained for 4 hours. 60 ml of ethyl acetate was added directly into each reaction liquid at the end of the reaction, stirred for another 1 hour, and filtered, so as to give micafungin diisopropylethylamine. The salt was dissolved in 30 ml of acetone and 30 ml of ethyl acetate, starching and filtered. Micafungin diisopropylethylamine was dried in vacuo to remove residual organic solvent. The purity and yield of Micafungin diisopropylethylamine determined by HPLC are shown in the following table.

| Hydrate | h | i | j | k | q | r |
|---|---|---|---|---|---|---|
| HPLC purity% | 97.49% | 97.02% | 97.03% | 97.57% | 97.46% | 96.88% |
| Yield% | 80.1% | 76.6% | 77.5% | 80.0% | 80.2% | 73.7% |

It can be concluded from the above comparative examples that, in Comparative Examples, the purity by HPLC and yield of the compound of formula II significantly decreased by using the hydrate of compound of formula I. However, compared with the hydrate of compound of formula I, the moisture content of which is controlled out of 8.0%-30%, the purity by HPLC and yield of the compound of formula II prepared from the hydrate of compound of formula I, the moisture content of which is controlled within 8.0%-30%, were better.

### Example 16

### Preparation of a pharmaceutical composition

| Hydrate B comprising the compound of formula I obtained in Example 2 | Lactose | anhydrous citric acid | NaOH |
|---|---|---|---|
| 2.5 g | 20 g | q.s. | q.s. |

20 g of lactose was dissolved into pure water (200 ml) at the temperature lower than 50°C. After cooling below 20°C, into the lactose solution was added 2.5 g of hydrate B comprising the compound of formula I obtained in Example 2. The resulting solution was gently stirred to avoid bubbles. 2% aqueous citric acid (0.95 ml)was added, and then into the solution was added 0.4% aqueous NaOH (approximately 24 ml) for adjusting pH to 5.5. And then the resulting solution was diluted with pure water to produce a given volume (250 ml). The resulting solution was filled into 100 vials (the volume of which is 10 ml), with 2.5 ml for each. The solution in each vial was lyophilized using the lyophilizer according to conventional methods, so as to obtain lyophilized compositions, with each containing 25 mg of the hydrate comprising compound of formula I.

The above examples are merely the preferred examples for the present invention, and such examples cannot be used to limit the scope of the invention. The substantial technical contents according to the present invention are broadly defined in the claims. And any entities or methods accomplished by others should be considered as the equivalents and fall within the scope as defined by the claims, if said entities or methods are the same as those defined by the claims.

## Claims

1. A hydrate of the compound of formula I, wherein R represents H or a cation capable of forming a pharmaceutically acceptable salt; wherein the mass percent of water in the hydrate is 8-30%;

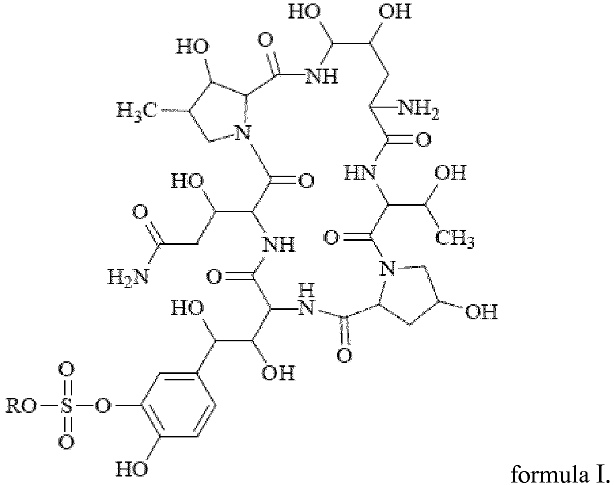

2. The hydrate according to claim 1, wherein the mass percent of water is 9.5-28.0%.

3. The hydrate according to claim 1 or 2, wherein the hydrate is prepared through the following steps:
(a) dissolving the compound of formula I into water or aqueous water-miscible organic solvent (i), and controlling pH of the solution comprising the compound of formula I;
(b) obtaining the hydrate comprising the compound of formula I by reducing the temperature and / or adding water-miscible organic solvent (i);
(c) obtaining the hydrate by centrifuging or filtrating;
(d) vacuum-drying the hydrate obtained in step (c) and controlling the mass percent of water in the solid within 8-30%.

4. The hydrate according to claim 3, wherein said organic solvent (i) is selected from C1-C4 lower alcohol.

5. The hydrate according to claim 4, wherein the lower alcohol is one or more selected from the group consisting of methanol, ethanol, n-propanol, and isopropanol.

6. A preparation method for the hydrate of compound of formula I, wherein the method includes the following steps:
(a) dissolving the compound of formula I into water or aqueous water-miscible organic solvent (i), and controlling pH of the solution comprising the compound of formula I;
(b) obtaining the hydrate comprising the compound of formula I by reducing the temperature and / or adding water-miscible organic solvent (i);
(c) obtaining the hydrate by centrifuging or filtrating;
(d) vacuum-drying the hydrate obtained in step (c) and controlling the mass percent of water in the solid within 8.0%-30%.

7. The preparation method according to claim 6, wherein said organic solvent (i) is selected from C1-C4 lower alcohol.

8. The preparation method according to claim 7, wherein the lower alcohol is one or more selected from the group consisting of methanol, ethanol, n-propanol, and isopropanol.

9. Use of the hydrate according to any one of claims 1-5 for preparing the compound of formula II

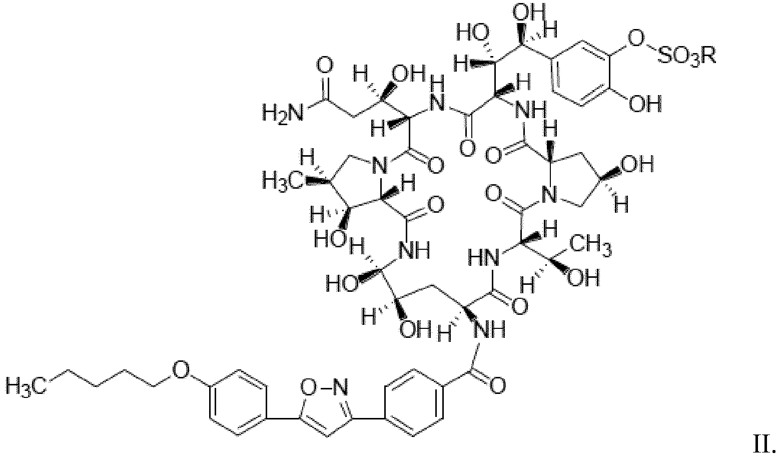

10. The hydrate according to any one of claims 1-5 for use in the treatment of fungal infections.

11. A method for the preparation of a pharmaceutical composition comprising the hydrate according to any one of claims 1-5 , the method including mixing the hydrate according to any one of claims 1-5 with a pharmaceutically acceptable carrier.

## Patentansprüche

1. Hydrat der Verbindung gemäß Formel I, wobei R H oder ein Kation, das in der Lage ist ein pharmazeutisch akzeptables Salz zu bilden, darstellt; wobei das Massenprozent von Wasser in dem Hydrat 8 - 30 % beträgt;

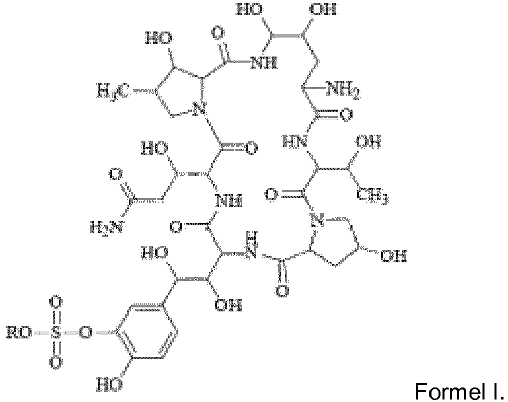

2. Hydrat gemäß Anspruch 1, wobei das Massenprozent von Wasser 9,5 - 28,0 % beträgt.

3. Hydrate gemäß Anspruch 1 oder 2, wobei das Hydrat im Verlauf der folgenden Schritte hergestellt wird:
(a) Lösen der Verbindung gemäß Formel I in Wasser oder einem wässrigen oder wassermischbaren organischen Lösungsmittel (i) und Steuern des pH-Wertes der Lösung, die die Verbindung gemäß Formel I umfasst;
(b) Erhalten des Hydrats, das die Verbindung gemäß Formel I umfasst, durch Verringern der Temperatur und/oder Hinzufügen eines wassermischbaren organischen Lösungsmittels (i);
(c) Erhalten des Hydrats durch Zentrifugieren oder Filtrieren;
(d) Vakuumtrocknen des in Schritt (c) erhaltenen Hydrats und Steuern des Massenprozents von Wasser in dem Lösungsmittel auf einen Wert zwischen 8 % und 30%.

4. Hydrat gemäß Anspruch 3, wobei das organische Lösungsmittel (i) ein niedrigmolekularer C1-C4-Alkohol ist.

5. Hydrat gemäß Anspruch 4, wobei der niedrigmolekulare Alkohol Methanol, Ethanol, n-Propanol und/oder Isopropanol ist.

6. Herstellungsverfahren für das Hydrat der Verbindung gemäß Formel I, wobei das Verfahren die folgenden Schritte umfasst:
(a) Lösen der Verbindung gemäß Formel I in Wasser oder einem wässrigen oder wassermischbaren organischen Lösungsmittel (i) und Steuern des pH-Wertes der Lösung, die die Verbindung gemäß Formel I umfasst;
(b) Erhalten des Hydrats, das die Verbindung gemäß Formel I umfasst, durch Verringern der Temperatur und/oder Hinzufügen eines wassermischbaren organischen Lösungsmittels (i);
(c) Erhalten des Hydrats durch Zentrifugieren oder Filtrieren;
(d) Vakuumtrocknen des in Schritt (c) erhaltenen Hydrats und Steuern des Massenprozents von Wasser in dem Lösungsmittel auf einen Wert zwischen 8 % und 30%.

7. Herstellungsverfahren gemäß Anspruch 6, wobei das organische Lösungsmittel (i) ein niedrigmolekularer C1-C4-Alkohol ist.

8. Herstellungsverfahren gemäß Anspruch 7, wobei der niedrigmolekulare Alkohol Methanol, Ethanol, n-Propanol und/oder Isopropanol ist.

9. Verwendung des Hydrats gemäß einem der Ansprüche 1 - 5 zum Herstellen der Verbindung gemäß Formel II

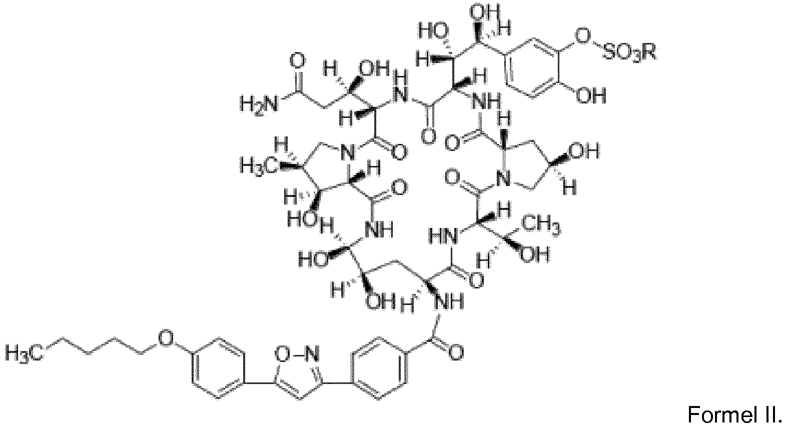

10. Hydrat gemäß einem der Ansprüche 1 - 5 zur Verwendung bei der Behandlung von Pilzinfektionen.

11. Verfahren zur Herstellung einer pharmazeutischen Zusammensetzung, die das Hydrat gemäß einem der Ansprüche 1 - 5 umfasst, wobei das Verfahren umfasst: Mischen des Hydrats gemäß einem der Ansprüche 1 - 5 mit einem pharmazeutisch akzeptablen Träger.

## Revendications

1. Hydrate du composé de formule I, dans lequel R représente H ou un cation capable de former un sel pharmaceutiquement acceptable ; où le pourcentage en poids d'eau dans l'hydrate est de 8 à 30 % ;

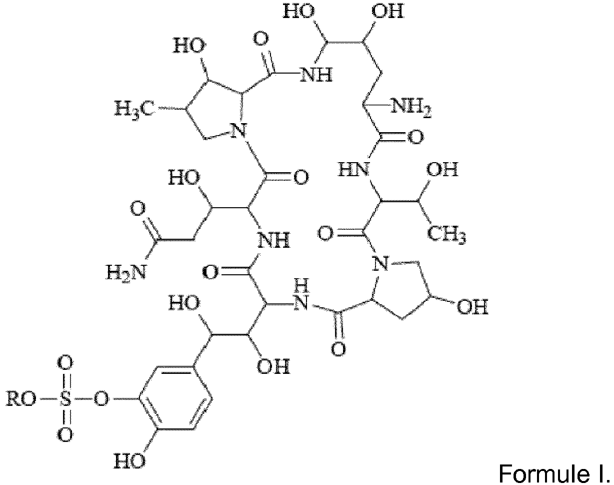

2. Hydrate selon la revendication 1, dans lequel le pourcentage en poids d'eau est de 9,5 à 28,0 %.

3. Hydrate selon la revendication 1 ou 2, dans lequel l'hydrate est préparé par les étapes suivantes :
(a) dissoudre le composé de formule I dans l'eau ou dans un solvant organique aqueux miscible à l'eau (i), et réguler le pH de la solution contenant le composé de formule I ;
(b) obtenir l'hydrate comprenant le composé de formule I par réduction de la température et/ou ajout de solvant organique miscible à l'eau (i) ;
(c) obtenir l'hydrate par centrifugation ou filtration ;
(d) sécher sous vide l'hydrate obtenu à l'étape (c) et contrôler le pourcentage en poids d'eau dans le solide dans la plage de 8-30 %.

4. Hydrate selon la revendication 3, dans lequel ledit solvant organique (i) est choisi parmi les alcools inférieurs en C1-C4.

5. Hydrate selon la revendication 4, dans lequel l'alcool inférieur est un ou plusieurs alcools choisis dans le groupe constitué par le méthanol, l'éthanol, le n-propanol et l'isopropanol.

6. Procédé de préparation de l'hydrate du composé de formule I, dans lequel le procédé comprend les étapes suivantes :
(a) dissoudre le composé de formule I dans l'eau ou dans un solvant organique aqueux miscible à l'eau (i), et réguler le pH de la solution contenant le composé de formule I ;
(b) obtenir l'hydrate comprenant le composé de formule I par réduction de la température et/ou ajout de solvant organique miscible à l'eau (i) ;
(c) obtenir l'hydrate par centrifugation ou filtration ;
(d) sécher sous vide l'hydrate obtenu à l'étape (c) et contrôler le pourcentage en poids d'eau dans le solide dans la plage de 8,0-30 %.

7. Procédé de préparation selon la revendication 6, dans lequel ledit solvant organique (i) est choisi parmi les alcools inférieurs en C1-C4.

8. Procédé de préparation selon la revendication 7, dans lequel ledit alcool inférieur est un ou plusieurs alcools choisis dans le groupe constitué par le méthanol, l'éthanol, le n-propanol et l'isopropanol.

9. Utilisation de l'hydrate selon l'une quelconque des revendications 1 à 5, pour préparer le composé de formule II

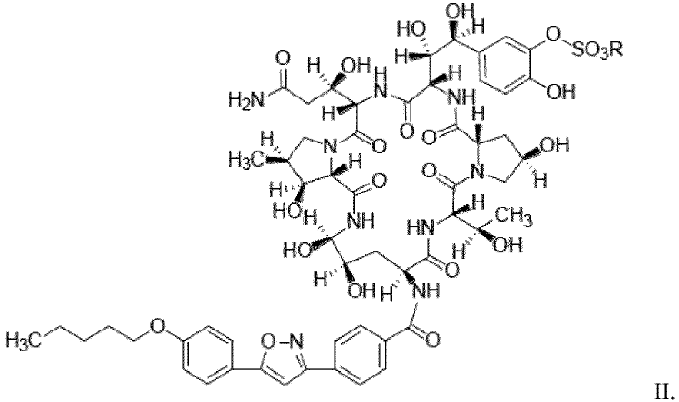

10. Hydrate selon l'une quelconque des revendications 1 à 5 pour son utilisation dans le traitement des infections fongiques.

11. Procédé de préparation d'une composition pharmaceutique comprenant l'hydrate selon l'une quelconque des revendications 1 à 5, le procédé comprenant le mélange de l'hydrate selon l'une quelconque des revendications 1 à 5 avec un véhicule pharmaceutiquement acceptable.
